(19) Europäisches Patentamt

European Patent Office

Office européen des brevets

(11) Publication number: **0 229 016 B1**

# EUROPEAN PATENT SPECIFICATION

(45) Date of publication of patent specification: **02.12.92**  (51) Int. Cl.⁵: **A61K  37/02**

(21) Application number: **87100067.5**

(22) Date of filing: **05.01.87**

(54) **Interleukin-2 compositions.**

(30) Priority: **07.01.86 JP 1846/86**

(43) Date of publication of application:
**15.07.87 Bulletin  87/29**

(45) Publication of the grant of the patent:
**02.12.92 Bulletin  92/49**

(84) Designated Contracting States:
**AT BE CH DE ES FR GB GR IT LI LU NL SE**

(56) References cited:
**EP-A- 0 158 487**
**EP-A- 0 217 645**
**WO-A-85/04328**
**US-A- 4 462 940**

(73) Proprietor: **SHIONOGI SEIYAKU KABUSHIKI KAISHA**
**12, Dosho-machi, 3-chome Higashi-ku,**
**Osaka-shi**
**Osaka 541(JP)**

Proprietor: **BIOGEN N.V.**
**15 Pietermaai**
**Willemstad Curacao, Netherlands**
**Antilles(NL)**

(72) Inventor: **Thatcher, David R.**
**64, Fairway Drive**
**Groton, Ma.(US)**
Inventor: **Shima, Kazuhiro**
**C-7-406, 3-1, Shinsenrinishimachi**
**Toyonaka-shi Osaka(JP)**

(74) Representative: **Vossius & Partner**
**Siebertstrasse 4 P.O. Box 86 07 67**
**W-8000 München 86(DE)**

Rank Xerox (UK) Business Services

**Description**

The present invention relates to interleukin-2 compositions very useful in medicine.

The so-called interleukin-2 compounds (hereinafter abbreviated to IL-2) are a group of proteins with T cell growth activity, NK cell (natural killer cell) growth activity, or similar activity. They are regarded as promising agents in the treatment of various cancer or immunodeficiency diseases, and more recently of acquired immune deficiency syndrome (AIDS).

Since IL-2 compounds are unstable in their usual preservation state, various methods for stabilization of the proteins have been proposed. For instance, papers have been published on the following: IL-2 compositions containing a reducing agent (JP-A-60-215631), IL-2 compositions containing human serum albumin (hereinafter abbreviated to HSA) and showing pH 3 to 6 in a state of solution (JP-A-60-222424), compositions of a biologically active substance combined with modified gelatin (JP-A-60-228422).

WO-A-8 504 328 discloses an interleukin-2 composition showing a pH of 6.1 to 9 in a state of solution and further comprising mannitol and SDS.

The present invention provides IL-2 compositions containing serum albumin and showing a pH of 6.1 to 9 in a state of solution, which are prepared by adjusting a solution of IL-2 to pH 8 - 11, more preferably pH 9 - 11 with a base and immediately thereafter to pH 6.1 - 9 with an acid; or by adjusting a solution of IL-2 to pH 2 - 6, more preferably pH 2 - 4 with an acid and immediately thereafter to pH 6.1 - 9 with a base.

As mentioned above, many attempts have been made to stabilize IL-2 and improve its solubilization.

IL-2 compounds are slightly soluble in water, especially in a near neutral condition, and their solubility is greatly decreased because of their aggregation at the isoelectric point (pH about 7.6). It has therefore been quite difficult to prepare neutral compositions of IL-2 because of problems in the preparation of freeze-dried compositions and the poor solubility of such compositions when reconstituted for practical use.

The compositions, however, are preferably prepared so as to be in a physiologically acceptable pH range, considering that they are injected intraarterially, intravenously, intramuscularly, subcutaneously or intracutaneously. It is presumed, for instance, that an acidic composition often irritates the injected site and may cause undesired topical reactions such as local pain or inflammation at the injected site. In order to avoid such undesired effects the present inventors have conducted research into new compositions and succeeded in preparing IL-2 compositions of the present invention which are adjusted at a pH value which is in the physiological range.

The compositions of the present invention exhibit excellent IL-2 stability in respect of the solution before freeze-drying and the freeze-dried compositions during preservation, in terms of the appearance and stability of the reconstituted solution, and in related aspects.

The present invention provides IL-2 compositions containing serum albumin (SA) and showing pH 6.1 - 9 in a state of solution, which are prepared by first adjusting a solution of IL-2 to pH 8 - 11, more preferably 9 - 11 and most preferably 9.5 - 10.5 with a base, and then immediately neutralizing it with an acid; or by adjusting said solution to pH 2 - 6, more preferably 2 - 4 and most preferably 2 - 3 with an acid, and then immediately neutralizing it with a base; and finally adjusting the solution so as to show pH 6.1 - 9, more preferably pH 6.1 - 8 and most preferably pH 6.5 - 7.5.

Any natural IL-2 can be used in this invention, and in addition recombinant human IL-2 is most preferably used.

IL-2 activity was assayed in the following manner in this invention:

IL-2 activity was assayed by determining the $^3$H-thymidine incorporation of CTLL-2 (IL-2 dependent murine cell line). The units of the IL-2 activity were determined as a reciprocal of the dilution at which 50% of the maximum counts per minute was observed, and were corrected in Jurkat Unit (hereinafter abbreviated to JU) using BRMP standard [Lot No. ISDP-841: obtained from the National Cancer Institute (NCI)] of Jurkat-derived IL-2 (Jurkat: human T cell leukaemia cell line). In the following Examples IL-2 having a specific activity of $(1.4\pm0.2)\times10^7$ JU/mg was employed, unless otherwise mentioned.

In this invention SA means serum albumin derived from a warm-blooded animal, including bovine serum albumin (hereinafter abbreviated to BSA), porcine serum albumin (PSA) and human serum albumin (HSA), from which a preferred one may be chosen according to the purpose. For instance, HSA is preferably employed in the preparation of compositions for human use. Addition of SA is useful in avoiding a decrease in IL-2 activity or in preventing IL-2 from being adsorbed onto the inner walls of vessels. SA may be used in 1 - 500 parts by weight, more preferably 10 - 200 parts by weight per 1 part of IL-2.

As to suitable bases in this invention, any kind of base can be employed as long as it is physiologically acceptable: as preferred examples alcoholamines such as N-methylglucamine, monoethanolamine, diethanolamine, and triethanolamine; alkylamines such as mono-, di-, and triethylamine; basic amino acids such as arginine, lysine, and histidine; inorganic bases such as sodium carbonate may be mentioned, and they may be used individually or as a mixture. In particular, N-methylglucamine, diethanolamine, triethanolamine, and arginine are preferred, being used individually or in combination. When an inorganic base is employed, it is appropriate to use in addition an amine or amide. The amount of base so used varies with the kind of base, but they may be generally used in 10 - 500 parts by weight, more preferably 50 - 200 parts by weight, per 1 part of IL-2.

As to suitable acids in this invention, any kind of acid may be employed as long as it is physiologically acceptable. For example, organic acids such as acetic acid, lactic acid, succinic acid, tartaric acid and citric acid; and inorganic acids such as hydrochloric acid and phoshoric acid may be mentioned. Additionally, physiologically acceptable salts may be used individually or in combination. Citric acid and tartaric acid are especially recommended. Although the amount of acid so used varies with the type of acid, they may be used in the amount needed to adjust the composition to a desirable pH value. They are generally used in an amount of 5 -250 parts by weight, more preferably 20 - 100 parts by weight, per 1 part of IL-2.

In this invention it is preferred to use sugars or sugar alcohols as the stabilizer or solubilizer for IL-2, or excipient. Sugar means monosaccharides, polysaccharides, or water-soluble glucans including fructose, glucose, mannose, sorbose, xylose, maltose, lactose, sucrose, dextran, pullulan, dextrin, cyclodextrin, soluble starch, hydroxyethyl starch and carboxymethylcellulose-Na. Of all these, maltose is the most preferred. Sugar alcohol mean $C_4$-$C_8$ and includes mannitol, sorbitol, inositol, dulucitol, xylitol and arabitol. Of all these, mannitol is the most preferred. The sugars or sugar alcohols mentioned above may be used individually or in combination. There is no fixed limit to the amount used, as long as it is soluble in the aqueous preparation. In practice they are used in an amount of 10 to 100 mg/ml.

It is preferred in this invention to use a buffer in order to minimize pH changes to the solution during the preparation steps or to the reconstituted solution. Any physiologically acceptable buffer can be used in this invention, and an amount of the buffer as necessary may be used to keep the solution at a desirable pH value. For example, phosphate-type or citrate-type buffers may be used.

Surfactants may be added to the solution. The addition of surfactants has a beneficial effect on the compositions of this invention, for example giving clearer solutions during preparation or on reconstitution, as well as lowering the adsorption rate of IL-2 onto the walls of vessels. Non-ionic surfactants are especially preferred for this purpose, e.g. polyoxyethylene (POE), hydrogenated castor oils, polyethylene glycols (PEG) including PEG300 and PEG400 and, polyoxyethylene sorbitan aliphatic esters. They are used in an amount of 0.1 mg to 1 mg per millilitre of the aqueous preparation.

An example of the preparation of the compositions of this invention is given below:
To a solution of HSA and maltose dissolved in distilled water is added IL-2, with a surfactant if desired. The solution thus prepared is adjusted to a pH of about 10 with diethanolamine, combined with a buffer, and then immediately adjusted to a pH of about 7 with citric acid. An appropriate amount of distilled water is added thereto to make a suitable quantity of the solution, which is then aseptically filtered, distributed into vials, and freeze-dried. In the foregoing procedures the entire amount of HSA may be used at once in one portion; or it may be divided into two portions and one may be added first and the other after adjusting to a pH of about 7.

When the resulting solution is finally adjusted to a pH of about 6 or above, IL-2 is kept stable, and the solutions are also kept clear during preparation and after reconstitution. Accordingly, there is no upper limit to the final pH value in this invention, but it would be at pH 6.1 - 9 for the preferred compositions. The compositions are preferably adjusted to a physiological pH value or thereabouts if they are intended as injections. In such a case the final pH value would be between 6.1 and 8, more preferably between 6.5 and 7.5. In an acidic range below the lowest limit mentioned above it is difficult to keep the appearance of the reconstituted solution clear. In an alkaline range over the upper limit it is possible to keep the compositions stable and to keep them clear when they are in solution, but hard to avoid irritation to the skin or blood vessels, and therefore such compositions are not as preferable. An IL-2 composition thus prepared as an aqueous solution can be used just as it is, but it is preferably first converted into a freeze-dried formulation in the following manner:
If a freeze-dried composition is needed, the following method would preferably be used:
Said aqueous solution in a vessel is cooled and immediately frozen to a temperature of -60 to -10°C, more preferably -40 to -25°C; the water is removed from the frozen product by sublimation to a preset moisture content at a reduced pressure between 0.005 and 1 mb for a period of 5 - 72 hours, supplying the heat consumed by sublimation, if required. The vessel is further filled with an inert gas such as nitrogen, or with dry air, and tightly sealed if necessary.

The present invention involves aqueous solutions as mentioned above, frozen or freeze-dried compositions of said solutions, and solutions of the freeze-dried compositions reconstituted in a suitable medium. These compositions of the invention have advantages in that the loss of IL-2 during the preparation steps and preservation is very slight, and the decrease in specific activity of IL-2 is minimal. In particular, the freeze-dried compositions have the significant advantage that the reconstituted solutions remain clear and adsorption of IL-2 onto the walls of the vessels is avoided. Furthermore, the compositions of this invention very rarely irritate the parts of the body where they are administered because they are prepared in a physiological pH range, and as a result they very rarely produce undesired effects locally such as pain from injection, inflammation at the injection sites, or the like.

The compositions of this invention are not limited in their route of application, but they may preferably be administered parenterally. When used as injections, they are administered intravenously, intramuscularly, subcutaneously or intracutaneously as a solution dissolved in distilled water, isotonic saline, or a suitable transfusion. As a matter of course, the compositions may be formulated into compositions for topical use such as an oral, nasal or otic administration together with a suitable carrier, excipient, or the like.

The present invention is explained in more detail by the following Examples and Experiments.

Example

(General procedures for preparing the aqueous preparation)

-- Method A --

To a solution of the entire amount or a portion of HSA dissolved in distilled water, if necessary together with a sugar or sugar alcohol, is added IL-2, if required with a surfactant. The resulting solution is adjusted to pH 8-11 with a base, and then to pH 6.1-9 with an acid after or before addition of a surfactant. The remaining amount of HSA, if any, is added thereto. The resulting solution may be filtered under aseptic conditions to give a sterile solution which may he distributed into vials for injection.

-- Method B --

To a solution of the entire amount or a portion of HSA dissolved in distilled water, if necessary together with a sugar or sugar alcohol, is added IL-2, if required with a surfactant. The resulting solution is adjusted to pH 2-6 with an acid, and then to pH 6.1-9 with a base after or before addition of a surfactant. The remaining amount of HSA, if any, is added thereto. The resulting solution may be filtered under aseptic conditions to give a sterile solution which may be distributed into vials for injection.

(General procedure for freeze-drying)

The above-prepared aqueous solution is rapidly cooled and frozen at -60 °C to -10 °C, preferably at -40 °C to -25 °C. The water is removed by sublimation to a preset moisture content at a pressure of 0.005-1 mb for a period of 5-72 hours, if necessary supplying the heat of sublimation. If necessary, the vessel may be charged with an inert gas such as nitrogen or with dry air and sealed.

Example 1

To a solution of 1.0 mg of HSA, 25 mg of mannitol and 0.1 mg of IL-2 in 1 ml of distilled water for injection use are added 10 mg of N-methylglucamine to bring the pH of the solution to about 10.1. Then the solution is adjusted to a pH of about 7.0 with 0.2 M monosodium tartrate buffer (about 1.2 ml as total amount).

The solution thus prepared is filtered through an appropriate membrane filter to give a sterile solution, which is distributed into vials and frozen at a temperature below -35 °C. The frozen composition is sublimated according to a conventional manner for freeze-drying, during which the composition placed in vials is kept at a temperature below -25°C, to give a freeze-dried composition.

Example 2

In a solution of 6.25 mg of HSA and 250 mg of maltose in 6.5 ml of distilled water for injection are dissolved 250 µg of IL-2. The solution is adjusted to a pH of about 10.5 with 27 mg of diethanolamine. 1.25 ml of 0.2 M sodium phosphate buffer (pH 6.9) and then 0.16 ml of 10% citric acid are added thereto to adjust the resulting solution to a pH of about 7.0, to which 6.25 mg of HSA and an appropriate amount of distilled water for injection are added to bring the volume to 10 ml. The resulting solution is filtered through

4

a membrane filter to give a sterile solution, which is distributed into vials of 2 ml each. They are frozen at a temperature below -35°C. The frozen composition placed in the vials is sublimated in a conventional manner for freeze-drying, during which the composition in vials is kept at a temperature below -25°C, to give a freeze-dried composition.

Example 3

In a solution of 6.25 mg of HSA and 250 mg of maltose in 6.5 ml of distilled water for injection are dissolved 250 μg of IL-2. The solution is adjusted to a pH of about 2.5 with 0.16 mg of 10% citric acid. 38.5 mg of triethanolamine and then 1.25 ml of 0.2 M sodium phosphate buffer (pH 6.9) are added thereto to adjust the solution to a pH of about 7.0, to which 6.25 mg of HSA and an appropriate amount of distilled water for injection are added to bring the volume to 10 ml. In the same manner as in Example 2, a freeze-dried composition is prepared.

Examples 4 to 30

In the same manner as explained above, the following compositions were prepared.

The amount of each component summarized in Table 1 corresponds to that used for preparing 1.2 ml or 2 ml of the solutions. The mark ○ means that the acid indicated by the mark is used in a calculated amount necessary to adjust the solution to the desirable pH value.

Table 1 (No. 1)

| Contents　　Example No. | | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|
| IL-2 (μg) | | 100 | 100 | 100 | 50 | 50 | 50 | 50 | 50 | 50 | 50 | 50 | 50 | 50 | 50 | 50 |
| HSA (mg) | | 1.0 | 1.0 | 1.0 | 2.5 | 2.5 | 2.5 | 2.5 | 2.5 | 2.5 | 2.5 | 2.5 | 5.0 | 2.5 | 2.5 | 2.5 |
| Base (mg) | N-methylglucamine | 5 | 5 | | 5 | 10 | 10 | 10 | | | | | | | | |
| | monoethanolamine | | | | | | | | | 3.1 | 3.1 | | | | | |
| | diethanolamine | | | | | | | | | | | 5.4 | 5.4 | 5.4 | 5.4 | 5.4 |
| | triethanolamine | | | | | | | | | | | | | | | |
| | arginine | | | 5 | | | | | 9.0 | | | | | | | |
| | lysine | | | | | | | | | | | | | | | |
| | sodium carbonate | | | | | | | | | | | | | | | |
| Acid (mg) | tartaric acid | | O | O | | | | | | | | | | | | |
| | citric acid | | | | O | O | | O | | O | | | O | O | O | O |
| | lactic acid | | | | | | O | | O | | O | O | | | | |
| | phosphoric acid | O | | | | | | | | | | | | | | |
| Sugar or Sugar-alcohol (mg) | glucose | | | | | | | 50 | | | | | | | | |
| | maltose | | | | 50 | 100 | 50 | | 50 | 50 | 50 | 50 | | 50 | 50 | 50 |
| | dextran | | | | | | | | | | | | 50 | | | |
| | mannitol | 50 | 50 | 50 | | | | | | | | | | | | |
| Others (mg) | polysorbate 80 | | | | | | | | | | | | | | | |
| | glycine | | | | | | | | | | | | | | | |
| | nicotinic acid | | | | | | | | | | | | | | | |
| Buffer (ml) | 0.2N phosphate buffer | 0.15 | | | 0.25 | 0.25 | 0.25 | 0.25 | 0.25 | 0.25 | 0.25 | 0.25 | 0.25 | 0.25 | 0.25 | 0.25 |
| | 0.5N tartarate buffer | | 0.12 | 0.12 | | | | | | | | | | | | |
| Preparation Method | | A | A | A | A | A | A | A | A | A | A | A | A | A | A | A |
| pH | | 7.0 | 7.0 | 7.0 | 7.0 | 7.0 | 7.0 | 7.0 | 7.0 | 7.0 | 7.0 | 7.0 | 7.0 | 7.5 | 7.9 | 9.0 |
| Amount of Unit Solution | | 1.2 | 1.2 | 1.2 | 2.0 | 2.0 | 2.0 | 2.0 | 2.0 | 2.0 | 2.0 | 2.0 | 2.0 | 2.0 | 2.0 | 2.0 |

EP 0 229 016 B1

Table 1 (No. 2)

| Contents Example No. | | 19 | 20 | 21 | 22 | 23 | 24 | 25 | 26 | 27 | 28 | 29 | 30 |
|---|---|---|---|---|---|---|---|---|---|---|---|---|---|
| IL-2 (µg) | | 50 | 50 | 50 | 50 | 50 | 50 | 50 | 50 | 50 | 50 | 50 | 50 |
| HSA (mg) | | 2.5 | 2.5 | 2.5 | 2.5 | 2.5 | 2.5 | 2.5 | 2.5 | 2.5 | 2.5 | 2.5 | 2.5 |
| Base (mg) | N-methylglucamine | | | | | | | | | | | | |
| | monoethanolamine | | | | | | | | | | | | |
| | diethanolamine | 5.4 | 5.4 | 5.4 | | | 10 | 5.4 | 5.4 | 10 | 5.4 | | |
| | triethanolamine | | | | 7.7 | 7.7 | | | | | | | |
| | arginine | | | | | | | | | | | | |
| | lysine | | | | | | | | | | | 2.7 | 7.5 |
| | sodium carbonate | | | | | | | | | | | | |
| Acid (mg) | tartaric acid | O | O | O | | | | | | | | | |
| | citric acid | | | | | | | | | | | O | O |
| | lactic acid | | | | O | O | O | O | O | O | O | | |
| | phosphoric acid | | | | | | | | | | | | |
| Sugar or Sugar-alcohol (mg) | glucose | | | | | | | | | | | | |
| | maltose | 50 | 50 | 50 | 50 | | 50 | 50 | 50 | 50 | 50 | 50 | 50 |
| | dextran | | | | | 25 | | | | | | | |
| | mannitol | | | | | | | | | | | | |
| Others (mg) | polysorbate 80 | | | | | | | | | | | | |
| | glycine | 0.50 | 10 | | | 0.25 | | | | | | 10 | |
| | nicotinic acid | | | | | | | | | | | | |
| Buffer (ml) | 0.2M phosphate buffer | 0.25 | 0.25 | 0.25 | 0.25 | 0.25 | 0.25 | 0.25 | 0.25 | 0.25 | 0.25 | 0.25 | 0.25 |
| | 0.5M tartarate buffer | | | | | | | | | | | | |
| Preparation Method | | A | A | A | A | A | A | A | A | B | B | A | A |
| pH | | 7.0 | 7.0 | 7.0 | 7.0 | 7.0 | 7.0 | 6.1 | 6.5 | 7.0 | 7.0 | 7.0 | 7.0 |
| Amount of Unit Solution | | 2.0 | 2.0 | 2.0 | 2.0 | 2.0 | 2.0 | 2.0 | 2.0 | 2.0 | 2.0 | 2.0 | 2.0 |

Experiment 1

Composition (A) was prepared as a reference by adding IL-2 to a solution which is preliminarily adjusted to neutral pH using both base and acid.

Composition (A)

A solution of 6.25 mg of HSA and 250 mg of maltose dissolved in 6.5 ml of distilled water for injection is adjusted to a pH of about 10.5 with 27 mg of diethanolamine, and then after addition of 1.25 ml of 0.2 M sodium phosphate buffer (pH 6.9) to a pH of about 7.0 with 0.16 ml of 10% citric acid. Then 6.25 mg of HSA is dissolved in the solution and 250 µg of IL-2 are added thereto. The final volume of the solution is brought to 10 ml with distilled water for injection.

Composition (A) is not practical because IL-2 became nearly insoluble in the course of the procedure outlined above.

Experiment 2

Compositions (B), (C), and (D) were prepared in order to elucidate how the final pH value of the solution influences the appearance of the reconstituted solution of a freeze-dried composition.

In the following Experiments, 1.0 ml of distilled water for injection was used for reconstitution of a freeze-dried composition.

Composition (B)

In a solution of 6.25 mg of HSA and 250 mg of maltose in 6.5 ml of distilled water for injection are dissolved 250 µg of IL-2. The solution is adjusted to a pH of about 10.1 with 27 mg of diethanolamine, and then to about pH 4.0 with 0.67 ml of 10% citric acid after addition of 1.25 ml of 0.2 M sodium phosphate buffer (pH 6.9), to which 6.25 mg of HSA are added. An appropriate amount of distilled water for injection is added thereto to bring the volume to 10 ml. The resulting solution is filtered through a membrane filter to give a sterile solution, which is distributed into vials of 2 ml each. They are frozen at a temperature below -35 °C. The frozen composition in the vials is sublimated according to a conventional manner for freeze-drying, during which the composition in vials is kept at a temperature below -25 °C, to give a freeze-dried composition (B).

Compositions (C) and (D)

In the same manner as in the preparation of the Composition (B), freeze-dried compositions (C) and (D) are prepared by freeze-drying the solutions adjusted to a pH of about 5.0 and about 5.6, respectively.

The appearance of both the reference compositions and the compositions of this invention was compared in the form of reconstituted solutions (Table 2).In Tables 2 to 4 the compositions are indicated by the numbers of the Examples. As is clearly seen from Table 2 the compositions prepared from the solutions showing a final pH of less than 6.1 were not preferable because of cloudiness of the reconstituted solutions.

Experiment 3

The appearance of the reconstituted solutions of the freeze-dried compositions of this invention is shown in Table 3.

The mark (-) or (±) used in Tables 2 to 4 means that the reconstituted solution was "clear" or "substantially clear". Additionally, the marks (+) to (+ + +) correspond to "slightly turbid" to "very turbid (turning cloudy)".

Table 2

| Composition | B | C | D | 2 5 | 2 6 | 2 | 1 6 | 1 7 | 1 8 |
|---|---|---|---|---|---|---|---|---|---|
| Final pH value | 4.0 | 5.0 | 5.6 | 6.1 | 6.5 | 7.0 | 7.5 | 7.9 | 9.0 |
| Turbidity when reconstituted | +++ | ++ | ++ | ± | ± | — | — | — | — |

EP 0 229 016 B1

Table 3 (No. 1)

| Composition | 1 | 2 | 3 | 4 | 5 | 6 | 7 | 8 | 9 |
|---|---|---|---|---|---|---|---|---|---|
| Final pH value | 7.0 | 7.0 | 7.0 | 7.0 | 7.0 | 7.0 | 7.0 | 7.0 | 7.0 |
| Turbidity when reconstituted | - | - | ± | ± | - | ± | - | - | - |

Table 3 (No. 2)

| Composition | 10 | 11 | 12 | 13 | 14 | 15 | 16 | 17 | 18 |
|---|---|---|---|---|---|---|---|---|---|
| Final pH value | 7.0 | 7.0 | 7.0 | 7.0 | 7.0 | 7.0 | 7.5 | 7.9 | 9.0 |
| Turbidity when reconstituted | ± | - | - | - | ± | + | - | - | - |

Table 3 (No. 3)

| Composition | 19 | 20 | 21 | 22 | 23 | 24 | 25 | 26 | 27 |
|---|---|---|---|---|---|---|---|---|---|
| Final pH value | 7.0 | 7.0 | 7.0 | 7.0 | 7.0 | 7.0 | 6.1 | 6.5 | 7.0 |
| Turbidity when reconstituted | − | − | ± | − | ± | − | ± | ± | ± |

Table 3 (No. 4)

| Composition | 28 | 29 | 30 |
|---|---|---|---|
| Final pH value | 7.0 | 7.0 | 7.0 |
| Turbidity when reconstituted | − | ± | ± |

Experiment 4

Accelerated stability tests were made on typical compositions of this invention. The results are shown in Table 4.

In the table, the item "Appearance" indicates any change observed in appearance of the freeze-dried compositions such as coloring, shrinking, caking, or the like; and "Turbidity when reconstituted" indicates any change observed in the appearance of the solutions of said compositions dissolved in 1 ml each of distilled water for injection. The Item "% Activity" means the remaining activity (%) when IL-2 activity of the freeze-dried compositions just prepared is regarded as 100%.

11

EP 0 229 016 B1

Table 4

| Composition | Stability — After 2 weeks at 40 °C | | |
|---|---|---|---|
| | Appearance | Turbidity | % Activity |
| 2 | No change | − | 110 % |
| 9 | No change | − | 94 % |
| 11 | No change | ± | 101 % |
| 13 | No change | − | 95 % |
| 14 | No change | − | 95 % |
| 24 | No change | − | 82 % |

**Claims**

**Claims for the following Contracting States : BE, CH, DE, FR, GB, IT, LI, LU, NL, SE**

1. An interleukin-2 composition containing serum albumin and having a pH of 6.1 to 9 when in solution which is prepared by adjusting a solution of interleukin-2 to a pH of 8 - 11 with a base and immediately thereafter to a pH of 6.1 - 9 with an acid; or by adjusting said solution to a pH of 2 - 6 with an acid and immediately thereafter to a pH of 6.1 - 9 with a base.

2. A composition as claimed in Claim 1, which is prepared by adjusting the solution of interleukin-2 to a pH of 9 - 11 with a base and immediately thereafter to a pH of 6.1 - 9 with an acid; or by adjusting said solution to a pH of 2 - 4 with an acid and immediately thereafter to a pH of 6.1 - 9 with a base.

3. A composition as claimed in Claim 1 or 2, wherein said serum albumin is human serum albumin.

**4.** A composition as claimed in Claim 3, further containing a sugar and/or sugar alcohol.

**5.** A composition as claimed in Claim 3 or 4, further containing a surfactant.

**6.** A composition as claimed in Claim 1 or 2, wherein (a) the base is N-methyl-glucamine, monoethanolamine, diethanolamine, triethanolamine or arginine, or a mixture thereof, and (b) the acid is citric acid, tartaric acid, lactic acid or phosphoric acid, or a mixture thereof.

**7.** A composition as claimed in any of Claims 1 to 6, in the form of a freeze-dried preparation.

**8.** A process for preparing interleukin-2 compositions comprising the steps of adjusting the pH value of a solution containing one weight-part of interleukin-2 and 1 to 500 weight-parts of serum albumin, optionally together with pharmaceutically acceptable carriers, to 8-11, preferably to 9-11 with a base and subsequently adjusting the pH value of the thus obtained basic solution immediately to 6.1-9 with an acid; or the steps of adjusting the pH value of said solution to 2-6, preferably to 2-4 with an acid and subsequently adjusting the pH value of the thus obtained acidic solution immediately to 6.1-9 with a base.

**9.** The process according to Claim 8, wherein said carrier is a sugar and/or sugar alcohol.

**10.** The process according to Claim 8, wherein said serum albumin is human serum albumin.

**11.** The process according to Claim 8, wherein said solution further contains a surfactant.

**12.** The process according to Claim 8, wherein said interleukin-2 is recombinant human interleukin-2.

**13.** The process according to Claim 8, wherein (a) the base is N-methyl-glucamine, monoethanolamine, diethanolamine, triethanolamine, or arginine or a mixture thereof; and (b) the acid is citric acid, tartaric acid, lactic acid, or phosphoric acid or a mixture thereof.

**Claims for the following Contracting States : AT, ES, GR**

**1.** A process for preparing an interleukin-2 composition containing serum albumin and optionally a pharmaceutically acceptable carrier and having a pH of 6.1 to 9 when in solution which comprises adjusting a solution of interleukin-2 to a pH of 8 - 11 with a base and immediately thereafter to a pH of 6.1 - 9 with an acid; or adjusting said solution to a pH of 2 - 6 with an acid and immediately thereafter to a pH of 6.1 - 9 with a base.

**2.** The process as claimed in Claim 1, which comprises adjusting the solution of interleukin-2 to a pH of 9 - 11 with a base and immediately thereafter to a pH of 6.1 - 9 with an acid; or adjusting said solution to a pH of 2 - 4 with an acid and immediately thereafter to a pH of 6.1 - 9 with a base.

**3.** The process according to claims 1 or 2 wherein the solution contains one weight-part of interleukin-2 and 1 to 500 weight-parts of serum albumin.

**4.** The process as claimed in any of Claims 1 to 3, wherein said serum albumin is human serum albumin.

**5.** The process according to any one of Claims 1 to 4, wherein said carrier is a sugar and/or sugar alcohol.

**6.** The process as claimed in any of Claims 1 to 5, wherein the composition further contains a surfactant.

**7.** The process as claimed in any of Claims 1 to 6, wherein (a) the base is N-methyl-glucamine, monoethanolamine, diethanolamine, triethanolamine or arginine, or a mixture thereof, and (b) the acid is citric acid, tartaric acid, lactic acid or phosphoric acid, or a mixture thereof.

**8.** The process according to any one of Claims 1 to 7, wherein said interleukin-2 is recombinant human interleukin-2.

9.  The process as claimed in any of Claims 1 to 8, further comprising the freeze-drying of the preparation.

**Patentansprüche**
**Patentansprüche für folgende Vertragsstaaten : BE, CH, DE, FR, GB, IT, LI, LU, NL, SE**

1.  Interleukin-2-Zusammensetzung, die Serumalbumin enthält und in Lösung einen pH-Wert von 6,1 bis 9 aufweist und die durch Einstellen einer Interleukin-2-Lösung mit einer Base auf einen pH-Wert von 8 bis 11 und sofort anschließend mit einer Säure auf einen pH-Wert von 6,1 bis 9 oder durch Einstellen der Lösung mit einer Säure auf einen pH-Wert von 2 bis 6 und sofort anschließend mit einer Base auf einen pH-Wert von 6,1 bis 9 hergestellt wird.

2.  Zusammensetzung nach Anspruch 1, die durch Einstellen der Interleukin-2-Lösung mit einer Base auf einen pH-Wert von 9 bis 11 und sofort anschließend mit einer Säure auf einen pH-Wert von 6,1 bis 9 oder durch Einstellen der Lösung mit einer Säure auf einen pH-Wert von 2 bis 4 und sofort anschließend mit einer Base auf einen pH-Wert von 6,1 bis 9 hergestellt wird.

3.  Zusammensetzung nach Anspruch 1 oder 2, wobei das Serumalbumin menschliches Serumalbumin ist.

4.  Zusammensetzung nach Anspruch 3, die weiterhin einen Zucker und/oder Zuckeralkohol enthält.

5.  Zusammensetzung nach Anspruch 3 oder 4, die weiterhin ein grenzflächenaktives Mittel enthält.

6.  Zusammensetzung nach Anspruch 1 oder 2, in der (a) die Base N-Methyl-glucamin, Monoethanolamin, Diethanolamin, Triethanolamin oder Arginin oder ein Gemisch davon ist und (b) die Säure Citronensäure, Weinsäure, Milchsäure oder Phosphorsäure oder ein Gemisch davon ist.

7.  Zusammensetzung nach einem der Ansprüche 1 bis 6 in der Form eines gefriergetrockneten Präparats.

8.  Verfahren zur Herstellung von Interleukin-2-Zusammensetzungen, wobei man die nachfolgenden Schritte durchführt: Einstellen des pH-Werts einer 1 Gewichtsteil Interleukin-2 und 1 bis 500 Gew.-Teile Serumalbumin, gegebenenfalls zusammen mit pharmazeutisch verträglichen Trägern, enthaltenden Lösung mit einer Base auf 8 bis 11, vorzugsweise 9 bis 11, und nachfolgendes sofortiges Einstellen auf 6,1 bis 9 mit einer Säure; oder Einstellen des pH-Werts der Lösung mit einer Säure auf 2 bis 6, vorzugsweise auf 2 bis 4, und nachfolgendes sofortiges Einstellen des pH-Werts der so erhaltenen sauren Lösung mit einer Base auf 6,1 bis 9.

9.  Verfahren nach Anspruch 8, wobei der Träger ein Zucker und/oder Zuckeralkohol ist.

10. Verfahren nach Anspruch 8, wobei das Serumalbumin menschliches Serumalbumin ist.

11. Verfahren nach Anspruch 8, wobei die Lösung weiterhin ein grenzflächenaktives Mittel enthält.

12. Verfahren nach Anspruch 8, wobei das Interleukin-2 rekombinantes menschliches Interleukin-2 ist.

13. Verfahren nach Anspruch 8, wobei (a) die Base N-Methyl-glucamin, Monoethanolamin, Diethanolamin, Triethanolamin oder Arginin oder ein Gemisch davon ist und (b) die Säure Citronensäure, Weinsäure, Milchsäure oder Phosphorsäure oder ein Gemisch davon ist.

**Patentansprüche für folgende Vertragsstaaten : AT, ES, GR**

1.  Verfahren zur Herstellung einer Interleukin-2-Zusammensetzung, die Serumalbumin und gegebenenfalls einen pharmazeutisch verträglichen Träger enthält und in Lösung einen pH-Wert von 6,1 bis 9 aufweist, wobei man eine Interleukin-2-Lösung mit einer Base auf einen pH-Wert von 8 bis 11 und sofort anschließend mit einer Säure auf einen pH-Wert von 6,1 bis 9 einstellt, oder die Lösung mit einer Säure auf einen pH-Wert von 2 bis 6 und sofort anschließend mit einer Base auf einen pH-Wert von 6,1 bis 9 einstellt.

2.  Verfahren nach Anspruch 1, wobei man die Interleukin-2-Lösung mit einer Base auf einen pH-Wert von

14

9 bis 11 und sofort anschließend mit einer Säure auf einen pH-Wert von 6,1 bis 9 einstellt, oder die Lösung mit einer Säure auf einen pH-Wert von 2 bis 4 und sofort anschließend mit einer Base auf einen pH-Wert von 6,1 bis 9 einstellt.

3. Verfahren nach Anspruch 1 oder 2, wobei die Lösung 1 Gew.-Teil Interleukin-2 und 1 bis 500 Gew.-Teile Serumalbumin enthält.

4. Verfahren nach einem der Ansprüche 1 bis 3, wobei das Serumalbumin menschliches Serumalbumin ist.

5. Verfahren nach einem der Ansprüche 1 bis 4, wobei der Träger ein Zucker und/oder Zuckeralkohol ist.

6. Verfahren nach einem der Ansprüche 1 bis 5, wobei die Zusammensetzung weiterhin ein grenzflächenaktives Mittel enthält.

7. Verfahren nach einem der Ansprüche 1 bis 6, wobei (a) die Base N-Methyl-glucamin, Monoethanolamin, Diethanolamin, Triethanolamin oder Arginin oder ein Gemisch davon ist, und (b) die Säure Citronensäure, Weinsäure, Milchsäure oder Phosphorsäure oder ein Gemisch davon ist.

8. Verfahren nach einem der Ansprüche 1 bis 7, wobei das Interleukin-2 rekombinantes menschliches Interleukin-2 ist.

9. Verfahren nach einem der Ansprüche 1 bis 8, wobei man das Präparat ferner gefriertrocknet.

**Revendications**
**Revendications pour les Etats contractants suivants : BE, CH, DE, FR, GB, IT, LI, LU, NL, SE**

1. Composition d'interleukine-2, contenant de l'albumine de sérum et ayant un pH de 6,1 à 9 lorsqu'elle est en solution, qui est préparée en réglant une solution d'interleukine-2 à un pH de 8-11 avec une base, et immédiatement après à un pH de 6,1-9 avec un acide, ou en réglant cette solution à un pH de 2-6 avec un acide et immédiatement après à un pH de 6,1-9 avec une base.

2. Composition suivant la revendication 1, qui est préparée en réglant la solution d'interleukine-2 à un pH de 9-11 avec une base et immédiatement après à un pH de 6,1-9 avec un acide, ou en réglant cette solution à un pH de 2-4 avec un acide et immédiatement après à un pH de 6,1-9 avec une base.

3. Composition suivant la revendication 1 ou 2, caractérisée en ce que l'albumine de sérum est une albumine de sérum humain.

4. Composition suivant la revendication 3, contenant en outre un sucre et/ou un alcool de sucre.

5. Composition suivant la revendication 3 ou 4, contenant en outre un agent tensioactif.

6. Composition suivant la revendication 1 ou 2, dans laquelle (a) la base est de la N-méthyl-glucamine, de la monoéthanolamine, de la diéthanolamine, de la triéthanolamine ou de l'arginine, ou un de leurs mélanges, et (b) l'acide est l'acide citrique, l'acide tartrique, l'acide lactique, ou l'acide phosphorique, ou un de leurs mélanges.

7. Composition suivant l'une quelconque des revendications 1 à 6, sous forme d'une préparation lyophilisée.

8. Procédé de préparation de compositions d'interleukine-2, comprenant les phases de réglage de la valeur du pH d'une solution contenant une partie en poids d'interleukine-2 et 1 à 500 parties en poids d'albumine de sérum, ainsi qu'éventuellement des supports pharmaceutiquement acceptables, à 8-11, de préférence à 9-11 avec une base, et de réglage ensuite de la valeur du pH de la solution basique ainsi obtenue, immédiatement à 6,1-9 avec un acide, ou bien une phase de réglage de la valeur du pH de la solution susdite à 2-6, de préférence à 2-4, avec un acide et ensuite réglage de la valeur du pH de la solution acide ainsi obtenue, immédiatement à 6,1-9 avec une base.

**9.** Procédé suivant la revendication 8, dans lequel le support susdit est un sucre et/ou un alcool de sucre.

**10.** Procédé suivant la revendication 8, dans lequel l'albumine de sérum susdite est une albumine de sérum humain.

**11.** Procédé suivant la revendication 8, dans lequel la solution susdite contient en outre un agent tensioactif.

**12.** Procédé suivant la revendication 8, dans lequel l'interleukine-2 précitée est une interleukine-2 humaine recombinante.

**13.** Procédé suivant la revendication 8, dans lequel (a) la base est de la N-méthyl-glucamine, de la monoéthanolamine, de la diéthanolamine, de la triéthanolamine ou de l'arginine, ou un de leurs mélanges, et (b) l'acide est l'acide citrique, l'acide tartrique, l'acide lactique ou l'acide phosphorique, ou un de leurs mélanges.

**Revendications pour les Etats contractants suivants : AT, ES, GR**

**1.** Procédé de préparation d'une composition d'interleukine-2, comprenant de l'albumine de sérum et éventuellement un support pharmaceutiquement acceptable, et ayant un pH de 6,1 à 9, lorsqu'elle est en solution, ce procédé comprenant le réglage d'une solution d'interleukine-2 à un pH de 8-11 avec une base, et immédiatement ensuite à un pH de 6,1-9 avec un acide, ou bien le réglage de cette solution à un pH de 2-6 avec un acide et immédiatement ensuite à un pH de 6,1-9 avec une base.

**2.** Procédé suivant la revendication 1, qui comprend le réglage de la solution d'interleukine-2 à un pH de 9-11 avec une base et immédiatement après à un pH de 6,1-9 avec un acide, ou le réglage de la solution susdite à un pH de 2-4 avec un acide et immédiatement après à un pH de 6,1-9 avec une base.

**3.** Procédé suivant la revendication 1 ou 2, dans lequel la solution contient une partie en poids d'interleukine-2 et 1 à 500 parties en poids d'albumine de sérum.

**4.** Procédé suivant l'une quelconque des revendications 1 à 3, dans lequel l'albumine de sérum est de l'albumine de sérum humain.

**5.** Procédé suivant l'une quelconque des revendications 1 à 4, dans lequel le support précité est un sucre et/ou un alcool de sucre.

**6.** Procédé suivant l'une quelconque des revendications 1 à 5, dans lequel la composition contient en outre un agent tensioactif.

**7.** Procédé suivant l'une quelconque des revendications 1 à 6, dans lequel (a) la base est de la N-méthyl-glucamine, de la monoéthanolamine, de la diéthanolamine, de la triéthanolamine ou de l'arginine, ou un de leurs mélanges, et (b) l'acide est l'acide citrique, l'acide tartrique, l'acide lactique ou l'acide phosphorique, ou un de leurs mélanges.

**8.** Procédé suivant l'une quelconque des revendications 1 à 7, dans lequel l'interleukine-2 est une interleukine-2 humaine recombinante.

**9.** Procédé suivant l'une quelconque des revendications 1 à 8, comprenant en outre la lyophilisation de la préparation.